# EUROPÄISCHE PATENTANMELDUNG

(11) **EP 4 620 424 A1**
(43) Veröffentlichungstag der Anmeldung: **24.09.2025**
(21) Anmeldenummer: 25163195.8
(22) Anmeldetag: 12.03.2025
(51) Int. Cl.: A61B 34/30, A61B 17/00

(54) **SCHNITTSTELLE ZUR VERBINDUNG EINER ANTRIEBSEINHEIT MIT EINEM MEDIZINISCHEN INSTRUMENT**

(30) Priorität: 19.03.2024 DE 102024107841
(71) Anmelder: Karl Storz SE & Co. KG, 78532 Tuttlingen (DE)
(72) Erfinder: Hoffmann, Martin, 13187 Berlin (DE); Klingels, Torben, 84034 Landshut (DE)

(57) **Zusammenfassung**

Die Erfindung betrifft eine Schnittstelle (10) zur Verbindung einer Antriebseinheit (12) mit einem medizinischen Instrument (14). Die Schnittstelle (10) umfasst wenigstens eine antriebsseitige Kopplungsvorrichtung (16) mit wenigstens einer antriebsseitigen Kopplungsfläche (18), und wenigstens eine zu der antriebsseitigen Kopplungsvorrichtung (16) komplementäre instrumentenseitigen Kopplungsvorrichtung (20) mit wenigstens einer instrumentenseitigen Kopplungsfläche (22). Die antriebsseitige und die instrumentenseitige Kopplungsvorrichtung (16, 20) sind unter Zwischenschaltung einer durchgängigen medizinischen Barriere (24) mechanisch miteinander koppelbar. Zumindest die antriebsseitige Kopplungsvorrichtung (16) umfasst wenigstens eine antriebsseitige Kolbenanordnung (40) mit wenigstens einem Kolben (26), welcher dazu eingerichtet ist, alternierend in entgegengesetzten Richtungen bewegt zu werden.

## Beschreibung

Die vorliegende Erfindung betrifft eine Schnittstelle zur Verbindung einer Antriebseinheit mit einem medizinischen Instrument, insbesondere als Teil eines medizinischen Robotersystems.

Medizinische Robotersysteme, insbesondere in Form von herkömmlichen medizinischen Master-Slave Robotersystemen, die beispielsweise für die mechanische Führung von medizinischen und/oder chirurgischen Instrumenten sowie die Steuerung derer Endeffektoren eingesetzt werden, weisen in der Regel eine lösbare Verbindung an einer Schnittstelle zwischen motorischen Antrieben auf der Roboterseite und den chirurgischen Instrumenten auf. Dabei werden die Antriebseinheit und das chirurgische Instrument meist über eine Getriebeanordnung kraftschlüssig miteinander gekoppelt.

Um Anforderungen an eine sterile Behandlungsumgebung erreichen zu können, ist im Bereich der Schnittstelle eine medizinische Barriere üblicherweise in Form einer Kunststofffolie vorgesehen, welche einen antriebsseitigen, in der Regel unsterilen, Teil der Schnittstelle von einem instrumentenseitigen sterilen Teil der Schnittstelle trennt bzw. abdeckt. Zur sterilen Kopplung einer Schnittstelle bzw. zur Übertragung zumindest einer Antriebskraft und/oder zumindest eines Antriebsdrehmoments zwischen den Komponenten der Schnittstelle, weisen bekannte medizinische Barrieren komplex geformte Folien oder speziell eingebettete Adapter in der Folie auf, über welche die Antriebskraft und/oder das Antriebsdrehmoment übertragbar sind. Derartige Schnittstellen bzw. dafür entwickelte medizinische Barrieren sind beispielsweise in den Dokumenten WO 2007/126443 A2 und US 2023/0390016 A1 beschrieben. Die komplex geformten Folien und/oder in die Folie integrierte Adapter verteuern die medizinische Barriere. Ferner müssen die Barrieren zudem punktgenau platziert werden, wodurch sich ein Arbeitsaufwand im Vorfeld eines medizinischen und/oder chirurgischen Eingriffs erhöht.

Ausgehend vom Stand der Technik liegt der Erfindung insbesondere aber nicht beschränkt darauf die Aufgabe zugrunde, eine Schnittstelle zur Verbindung einer Antriebseinheit mit einem medizinischen Instrument vorteilhaft weiterzuentwickeln, insbesondere hinsichtlich von Kosten und/oder einer Installation bzw. Deinstallation.

Ferner ist es insbesondere eine Aufgabe der vorliegenden Erfindung, eine zuverlässige und funktionsfähige Schnittstelle mit optimierten Eigenschaften bereitzustellen, sodass insbesondere vor einem medizinischen Eingriff und/oder während eines medizinischen Eingriffs an einem Patienten stets ein steriles Arbeitsfeld gewährleistet bzw. sichergestellt werden kann.

Diese Aufgabe wird erfindungsgemäß durch die Merkmale der unabhängigen Ansprüche gelöst. Weiterbildungen der Erfindung sind den abhängigen Ansprüchen zu entnehmen.

Die Erfindung betrifft eine Schnittstelle zur Verbindung einer Antriebseinheit mit einem medizinischen Instrument umfassend:
- wenigstens eine antriebsseitige Kopplungsanordnung mit wenigstens einer antriebsseitigen Kopplungsfläche, und
- wenigstens eine zu der antriebsseitigen Kopplungsvorrichtung komplementären instrumentenseitigen Kopplungsvorrichtung mit wenigstens einer instrumentenseitigen Kopplungsfläche,

wobei die antriebsseitige und die instrumentenseitige Kopplungsvorrichtung unter Zwischenschaltung einer durchgängigen medizinischen Barriere mechanisch miteinander koppelbar sind, und
wobei zumindest die antriebsseitige Kopplungsvorrichtung wenigstens eine Kolbenanordnung mit wenigstens einem Kolben umfasst, welcher dazu eingerichtet ist, alternierend in entgegengesetzten Richtungen bewegt zu werden.

Durch eine derartige Ausgestaltung kann eine vorteilhaft weiterentwickelte Schnittstelle bereitgestellt werden. Insbesondere kann eine Installation bzw. Deinstallation medizinischer Instrumente vereinfacht und es können Kosten sowie Stillstandszeiten reduziert werden, da eine durchgängige medizinische Barriere zum Einsatz kommen kann, die insbesondere "einfach geformt ist und/oder auf speziell eingebettete Adapter zur Übertragung einer Antriebskraft und/oder eines Antriebsdrehmoments verzichten kann. Hierdurch kann eine zeitaufwändige präzise Platzierung der medizinischen Barriere entfallen. Zudem kann ein Kostenaufwand für jeden medizinischen Einsatz reduziert werden, da eine nur einmalig verwendbare medizinische Barriere möglichst kostengünstig gehalten werden kann. Insbesondere kann eine medizinische Standardbarriere zum Einsatz kommen. Ferner kann durch die erfindungsgemäßen Merkmale eine besonders zuverlässige und funktionsfähige Schnittstelle mit optimierten Eigenschaften bereitgestellt werden, sodass insbesondere vor einem medizinischen Eingriff und/oder während eines medizinischen Eingriffs an einem Patienten stets ein steriles Arbeitsfeld gewährleistet und eine zuverlässige mechanische Kopplung sichergestellt ist.

Die Schnittstelle kann Teil eines medizinischen Robotersystems sein. Das Robotersystem kann zumindest einen Roboterarm, eine Bedienkonsole, einen Elektronikschrank, insbesondere ein Elektronik-Rack, beispielsweise zur Aufnahme zusätzlicher Geräte wie eines HF-Generators, eine Anzeigeeinheit, eine Patientenliege, eine Aufbewahrungseinheit für verschiedene medizinische Instrumente zur Verwendung mit der Schnittstelle und/oder weitere, dem Fachmann als sinnvoll erscheinende Einheiten aufweisen. Die durchgängige medizinische Barriere kann ebenfalls Teil des medizinischen Robotersystems sein. **In** einem Betriebszustand kann die Schnittstelle durch den Roboterarm getragen und durch diesen bewegbar sein.

Bei dem medizinischen Instrument kann es sich um einen beliebigen, dem Fachmann als sinnvoll erscheinenden Effektor, insbesondere in Form eines medizinischen und/oder chirurgischen Instruments handeln, welches bevorzugt durch den Roboterarm getragen und durch diesen bewegbar sein kann. Das medizinische Instrument kann mit der instrumentenseitigen Kopplungsvorrichtung fest oder lösbar verbunden sein oder Teil der instrumentenseitigen Kopplungsvorrichtung sein. Das medizinische Instrument kann beispielsweise eine laparoskopische Einheit, ein Endoskop, ein Mikroskop, ein Exoskop, ein Skalpell, einen Bohrer, einen Schaber, eine Klammer, eine Zange, eine Schere, eine Spritze, einen Katheder und/oder weitere, dem Fachmann als sinnvoll erscheinende Einheiten aufweisen, welche mittels der Antriebseinheit über die Schnittstelle antreibbar und/oder ansteuerbar sein können.

In dem gekoppelten Zustand der Schnittstelle kontaktiert die antriebsseitige Kopplungsfläche eine erste Seite der medizinischen Barriere und die instrumentenseitige Kopplungsfläche eine der ersten Seite gegenüberliegende zweite Seite der medizinischen Barriere. Die Kopplungsflächen können dabei bevorzugt zueinander komplementär ausgestaltet sein, insbesondere um eine formschlüssige Aufnahme und/oder ein formschlüssiges Einklemmen der medizinischen Barriere zwischen den Verbindungselementen zu ermöglichen. Die Kopplungsflächen können jeweils eine Gesamtfläche von zumindest 50 mm², insbesondere von zumindest 100 mm² und beispielsweise von zumindest 250 mm² aufweisen.

Bei einer "Kopplungsvorrichtung" kann es sich insbesondere um eine mechanische Einrichtung handeln, die dazu eingerichtet ist, zwei oder mehrere Bauteilkomponenten miteinander zu verbinden und/oder mit ein oder mehreren Bauteilkomponenten gekoppelt zu werden. Die Kopplungsvorrichtung kann dazu eingerichtet sein, eine feste Verbindung zwischen den Bauteilkomponenten herzustellen, sodass diese miteinander interagieren können, sei es mechanisch, elektrisch, hydraulisch und/oder auf eine andere Weise. Die Kopplungsvorrichtungen können mit ihren Kopplungsflächen zusammengesetzt einen um eine Drehsymmetrieachse zumindest teilweise rotationssymmetrischen, insbesondere zumindest teilweise kreiszylindrischen und/oder wenigstens teilweise kegelstumpfförmigen, Körper ausbilden. Die Kupplungsvorrichtungen können dabei zumindest teilweise quer zur Drehsymmetrieachse zusammengesetzt sein. In manchen Ausgestaltungen kann die antriebsseitige Kopplungsvorrichtung größer ausgestaltet sein als die instrumentenseitige Kopplungsvorrichtung. Die antriebsseitige Kopplungsvorrichtung kann einen Aufnahmeraum zur Aufnahme der instrumentenseitigen Kopplungsvorrichtung aufweisen.

Unter "eingerichtet" soll insbesondere speziell programmiert, vorgesehen, ausgelegt, ausgebildet und/oder ausgestattet verstanden werden. Darunter, dass ein Objekt zu einer bestimmten Funktion eingerichtet ist, soll ferner verstanden werden, dass das Objekt diese bestimmte Funktion in zumindest einem Anwendungs- oder Betriebszustand erfüllt oder ausführt.

Die durchgängige medizinische Barriere ist frei von Öffnungen, Einlegern und/oder speziellen eingebetteten Adaptern zur Übertragung einer Antriebskraft und/oder eines Antriebsmoments. Die durchgängige medizinische Barriere kann über Ihre gesamte Flächenerstreckung eine Dicke von höchstens 1 mm, insbesondere von maximal 0,5 mm und beispielsweise von höchstens 0,1 mm aufweisen. Die durchgängige medizinische Barriere ist bevorzugt flexibel ausgebildet. Besonders bevorzugt ist die durchgängige medizinische Barriere als ein entsprechend bearbeitetes und/oder beschichtetes Gewebe und/oder vorzugsweise als eine Folie, insbesondere als eine Kunststofffolie, ausgebildet.

Dass die antriebsseitige und die instrumentenseitige Kopplungsvorrichtung mechanisch miteinander koppelbar ist, soll im Rahmen der vorliegenden Erfindung insbesondere so verstanden werden, dass die antriebsseitige und die instrumentenseitige Kopplungsvorrichtung dazu eingerichtet sind, verliersicher und/oder funktional miteinander verbunden zu werden, um eine bestimmte Funktion zu ermöglichen. Durch die mechanische Kopplung bzw. durch die Verbindung können Kräfte und/oder Bewegungen zwischen den beiden Kopplungsvorrichtungen direktional oder bidirektional übertragen werden, was es ermöglicht, dass die beiden Kopplungsvorrichtungen zusammenarbeiten bzw. sich gegenseitig beeinflussen.

Die antriebsseitige Kopplungsvorrichtung und/oder die instrumentenseitige Kopplungsvorrichtung können ein Gehäuse umfassen. Das Gehäuse kann insbesondere dazu eingerichtet sein, Komponenten, die zumindest abschnittsweise von dem Gehäuse umgeben werden, gegen äußere Einflüsse wie Staub, Feuchtigkeit und/oder mechanische Beschädigungen zu schützen. Das Gehäuse kann aus verschiedenen Materialien wie Kunststoff, Metall oder Gummi bestehen. Das Gehäuse kann so konfiguriert sein, dass es einfach mit wenigstens einem anderen Gehäuse oder System verliersicher verbunden werden kann.

Die antriebsseitige Kopplungsvorrichtung umfasst erfindungsgemäß wenigstens eine Kolbenanordnung. Die wenigstens eine Kolbenanordnung kann insbesondere in einem antriebsseitigen Gehäuse angeordnet sein. Unter einer "Kolbenanordnung" soll insbesondere die Positionierung und/oder Anordnung von Kolben in der wenigstens einen antriebsseitigen Kopplungsvorrichtung verstanden werden. Bei einem Kolben kann es sich insbesondere um ein Bauteil, vorzugsweise ein zylindrisches Bauteil handeln, das zumindest teilweise oder vollständig entlang einer Bezugsachse bewegbar ist. In einigen Ausführungsformen kann der wenigstens eine Kolben auch als eine Fluid- und/oder Gassäule ausgebildet sein, die dazu eingerichtet ist, hydraulisch und/oder pneumatisch verformt zu werden. Vorzugsweise fällt die Bezugsachse, auf welcher der Kolben bewegbar ist, mit dessen Längsachse zusammen. Der Kolben kann Teil eines hydraulischen, pneumatischen oder anderen vorteilhaften mechanischen Systems sein. Insbesondere ist der wenigstens eine Kolben dazu eingerichtet, alternierend in entgegengesetzten Richtungen bewegt zu werden. Vorzugsweise ist der wenigstens eine Kolben dazu eingerichtet, entlang der Bezugsachse in einer Kolbenbewegung hin und her, insbesondere auf und ab, bewegt zu werden. Die Kolbenbewegung kann insbesondere aperiodisch sein.

Die Bewegung des wenigstens einen Kolbens kann insbesondere entlang einer Bezugsachse erfolgen, die schräg oder senkrecht zu der antriebsseitigen Kopplungsfläche verläuft. In anderen Worten kann die Bezugsachse, entlang welcher der wenigstens eine Kolben bewegbar sein kann nicht parallel zu der antriebsseitigen Kopplungsfläche verlaufen.

Gemäß einer Weiterbildung kann die Kolbenanordnung wenigstens einen weiteren Kolben umfassen, welcher mit dem wenigstens einen Kolben interdependent gekoppelt ist. "Interdependent gekoppelt" soll insbesondere bedeuten, dass der wenigstens eine Kolben derart mit dem wenigstens einen weiteren Kolben gekoppelt sein kann, dass sich die Kolben insbesondere hinsichtlich ihrer Position gegenseitig beeinflussen. Dabei ist die Position und/oder das Verhalten bzw. die Funktionsweise eines jeden Kolbens von dem oder den innerhalb einer Kopplungsanordnung anderen gekoppelten Kolben abhängig. Hinsichtlich des Bewegungsverhaltens zweier interdependent miteinander gekoppelter Kolben bedeutet das, dass deren Bewegungen zueinander entgegengesetzt sind. In anderen Worten bewegt sich der wenigstens eine Kolben in eine erste Richtung, wenn sich der interdependent mit dem wenigstens einen Kolben gekoppelte weitere Kolben in eine zweite Richtung bewegt, die der ersten Richtung entgegengesetzt ist und umgekehrt. Die Bewegung des wenigstens einen Kolbens kann dabei eine Bewegung des Instruments regeln und die Bewegung des wenigstens einen weiteren Kolbens kann entsprechend die entgegengesetzte Bewegung des Instruments regeln.

In einigen Ausführungsformen kann die Schnittstelle der eingangsbezeichneten Art wenigstens eine Hebelvorrichtung umfassen. Dabei kann der wenigstens eine Kolben zusammen mit dem wenigstens einen weiteren Kolben über die wenigstens eine Hebelvorrichtung aktuierbar sein. Bei der Hebelvorrichtung kann es sich insbesondere um einen Kipphebel handeln, der dazu eingerichtet ist, eine lineare Bewegung des Kolbens bzw. der Kolben herbeizuführen. Die Hebelvorrichtung kann wenigstens ein erstes Hebelende und wenigstens ein zweites Hebelende umfassen. Die Hebelenden können um einen Hebelpunkt rotierbar sein. Auf jedem dieser Hebelenden kann ein Kolben angeordnet sein. Durch Verlagern der Hebelvorrichtung bzw. der Hebelenden um den Hebelpunkt, können auf den Hebelenden angeordnete Kolben verlagert werden. Insbesondere kann die Hebelvorrichtung derart konfiguriert sein, dass sich die Hebelenden in entgegengesetzte Raumrichtungen erstrecken. Der wenigstens eine Kolben und/oder der wenigstens eine weitere Kolben kann eine Unterseite umfassen, an der er mit der Hebelvorrichtung derart gekoppelt ist, dass dessen Ausrichtung im Bezug zu der Hebelvorrichtung insbesondere während einer Bewegung bzw. Verkippung der Hebelvorrichtung variierbar ist. Der wenigstens eine Kolben und/oder der wenigstens eine weitere Kolben können in einigen Ausführungsformen gelenkig mit der wenigstens einen Hebelvorrichtung bzw. deren Enden gekoppelt sein. Der wenigstens eine Kolben und/oder der wenigstens eine weitere Kolben können die jeweiligen Enden der Hebelvorrichtung kontaktieren.

Gemäß einer weiteren Ausführungsform kann die wenigstens eine Kolbenanordnung wenigstens eine Zylinderführung umfassen, welche dazu eingerichtet ist, den wenigstens einen Kolben und/oder den wenigstens einen weiteren Kolben während einer Bewegung des Kolbens entlang einer Führungsachse zu führen. Bei einer Zylinderführung kann es sich insbesondere um eine mechanische Komponente handeln, die dazu eingerichtet ist, die Bewegung eines Kolbens entlang einer vorteilhaften bzw. definierten Führungsstrecke zu führen und/oder zu stabilisieren. Insbesondere kann die Zylinderführung dazu eingerichtet sein, eine axiale Bewegung eines darin geführten Kolbens zu gestatten und gleichzeitig eine seitliche Bewegung zu verhindern und/oder zu begrenzen. Die Zylinderführung kann als ein Hohlzylinder ausgebildet sein. Die Form der Zylinderführung kann je nach Form bzw. Kontur des zu führenden Kolbens variieren. In einigen Ausführungsformen kann die wenigstens eine Zylinderführung in Form wenigstens einer Materialaussparung bzw. Durchgangsbohrung in einem monolithischen Strukturelement ausgebildet sein.

In einer weiteren Ausführungsform kann die wenigstens eine Zylinderführung wenigstens eine Kante aufweisen, welche zumindest abschnittsweise und bevorzugt vollständig abgerundet ist. Ferner kann der wenigstens eine Kolben und/oder der wenigstens eine weitere Kolben wenigstens eine Stirnfläche aufweisen, dessen Kante zumindest abschnittsweise, bevorzugt vollständig abgerundet ist. Stirnflächen sollen hierin als die Flächen verstanden werden, die im gekoppelten Zustand zu der zwischengeschalteten medizinischen Barriere gerichtet sind.

Abgerundete Kanten weisen insbesondere den Vorteil auf, Beschädigungen und/oder Verschleiß innerhalb der Schnittstelle zu verhindern oder zumindest zu minimieren. Insbesondere können Beschädigungen der medizinischen Barriere und damit potenziell verbundene Kontaminationen vorteilhaft vermieden werden.

Gemäß einer Weiterbildung umfasst die Kolbenanordnung wenigstens eine Federvorrichtung, welche dazu eingerichtet ist, den wenigstens einen Kolben und/oder den wenigstens einen weiteren Kolben in einer federbelasteten Position axial vorzuspannen, um eine zuverlässige Bewegungsübertragung auf ein medizinisches Instrument zu übertragen. Die Federvorrichtung kann zwischen einem Kolben und der Hebelvorrichtung angeordnet sein. In einigen Ausführungsformen kann die Federvorrichtung auch innerhalb des wenigstens einen Kolbens und/oder des wenigstens einen weiteren Kolbens angeordnet sein. Die Federvorrichtung kann insbesondere dazu eingerichtet sein, eine Vorspannkraft zu erzeugen, die den vorzuspannenden Kolben in eine bestimmte Position und/oder gegen eine andere Komponente insbesondere einen komplementären Kolben drückt. Dadurch kann ein ständiger Kontakt zwischen zwei interagierenden Komponenten, insbesondere zwei interagierenden Kolben, erreicht und die zuverlässige Funktionsweise sichergestellt werden. Die Federvorrichtung kann insbesondere eine metallische und//oder elastomere Schraubenfeder, eine hydraulische Feder und/oder eine pneumatische Feder umfassen.

In einigen Ausführungsformen kann die Schnittstelle der eingangsbezeichneten Art eine Hubvorrichtung umfassen, welche dazu eingerichtet ist, zumindest die antriebsseitige Kolbenvorrichtung und/oder die antriebsseitige Kopplungsvorrichtung funktionsfähig mit der instrumentenseitigen Kopplungsvorrichtung zu koppeln. Eine funktionsfähige Kopplung kann erfolgen, indem die antriebsseitige Kopplungsvorrichtung und/oder die instrumentenseitige Kopplungsvorrichtung mittels einer oder mehrerer Hubvorrichtungen, bevorzugt automatisiert in Richtung der jeweils anderen Kopplungsvorrichtung verlagert und/oder mit dieser in Eingriff gebracht wird. Unter einer Hubvorrichtung soll hierin insbesondere eine mechanische, hydraulische und/oder pneumatische Einrichtung verstanden werden, die eine vorzugsweise automatisierte Kopplung oder Entkopplung der antriebsseitigen Kopplungsvorrichtung mit der instrumentenseitigen Kopplungsvorrichtung ermöglicht. Mittels einer derartigen Hubvorrichtung können präzise Bewegungen ausgeführt werden. Gemäß einigen Ausführungsformen kann die Hubvorrichtung dazu eingerichtet sein, zumindest wenigstens eine Kolbenanordnung relativ zum antriebsseitigen Gehäuse zu verlagern und/oder in einer vorbestimmten Position reversibel zu arretieren. Auf diese Weise kann nicht nur die Kopplung bzw. Entkopplung der Schnittstelle vereinfacht, sondern auch Fehler bzw. Schäden aufgrund fehlerhafter Montage verhindert oder zumindest ein solches Risiko verringert werden.

Gemäß einer Weiterbildung kann die antriebsseitige Kopplungsvorrichtung eine Mehrzahl an Kolbenanordnungen umfassen. Jeder Kolbenanordnung kann ein Freiheitsgrad des medizinischen Instruments zugeordnet sein. In anderen Worten kann sich jede Kolbenanordnung unabhängig von anderen Kolbenanordnungen bewegen. Der Freiheitsgrad bezieht sich dabei auf die Anzahl der unabhängigen Bewegungen, die das medizinische Instrument ermöglicht. Vorzugsweise sind die Kolbenanordnungen nebeneinander angeordnet. Die Kolbenanordnungen können insbesondere platzsparend positioniert sein, sodass sie nur wenig Bauraum einnehmen.

In einigen Ausführungsformen kann die antriebsseitige Kopplungsvorrichtung wenigstens eine Antriebseinheit umfassen, welche dazu eingerichtet ist, die wenigstens eine Kolbenanordnung bzw. Hebelvorrichtung anzutreiben und/oder zu bewegen. Die Antriebseinheit kann dazu eingerichtet sein, zumindest eine Antriebskraft und/oder zumindest ein Antriebsdrehmoment auf die Kolbenanordnung und/oder die Hebelvorrichtung zu übertragen. Vorzugsweise kann jede Hebelvorrichtung mit einer jeweiligen Antriebseinheit gekoppelt sein. Die Antriebseinheit kann wenigstens eine Antriebswelle, wenigstens ein Zahnradgetriebe, wenigstens einen Riemenantrieb, wenigstens einen Seiltrieb, wenigstens einen Kettenantrieb und/oder wenigstens ein Zahnstangengetriebe umfassen. Ferner kann die Antriebseinheit wenigstens einen Elektromagneten, wenigstens einen Piezoaktor, wenigstens einen hydraulischen Aktuator und/oder wenigstens einen pneumatischen Aktuator umfassen. In einer alternativen Ausführungsform kann jeder Kolben bzw. jede Kolbenanordnung mit einer jeweiligen Antriebseinheit gekoppelt sein. Die Antriebseinheit kann in dem gekoppelten Zustand von einer Roboterseite ansteuerbar sein.

Gemäß einer Weiterbildung kann die wenigstens eine instrumentenseitige Kopplungsvorrichtung wenigstens eine zu der wenigstens einen antriebsseitigen Kolbenanordnung komplementäre Kolbenanordnung umfassen, die dazu eingerichtet ist, Bewegungen der wenigstens einen antriebsseitigen Kolbenanordnung aufzunehmen und/oder Bewegungen auf die wenigstens eine antriebsseitigen Kolbenanordnung zu übertragen. Die wenigstens eine komplementäre Kolbenanordnung kann in einem instrumentenseitigen Gehäuse angeordnet sein. Die komplementäre Kolbenanordnung kann wenigstens einen komplementären Kolben und wenigstens einen weiteren komplementären Kolben umfassen.

Die Übertragung der Bewegungen kann abgesehen von der zwischengeschalteten medizinischen Barriere direkt von einem antriebsseitigen Kolben auf einen instrumentenseitigen Kolben oder umgekehrt und/oder indirekt erfolgen, indem beispielsweise die Kolben insbesondere hydraulische und/oder pneumatische Systeme betätigen, die miteinander interagieren.

Um Beschädigungen und/oder Verschleiß innerhalb der Schnittstelle zu verhindern oder zumindest zu minimieren, kann die wenigstens eine komplementäre Kolbenanordnung wenigstens eine Kante aufweisen, welche zumindest abschnittsweise, bevorzugt vollständig, abgerundet ist. Insbesondere können mittels einer derartigen Ausgestaltung Beschädigungen der medizinischen Barriere und damit potenziell verbundene Kontaminationen vorteilhaft vermieden werden.

Gemäß einer Weiterbildung kann die Kolbenvorrichtung wenigstens eine elastische Membran umfassen, die dazu eingerichtet ist, zumindest abschnittsweise die Stirnfläche eines Kolbens zu bedecken. Eine derartige Ausgestaltung senkt das Risiko einer Beschädigung der medizinischen Barriere, da sie verhindert, dass die medizinische Barriere etwa zwischen dem Kolben und dessen Zylinderführung eingeklemmt wird. Aufgrund der Elastizität der Membran, kann diese mechanisch, hydraulisch und/oder pneumatisch verformt bzw. ausgewölbt und/oder eingedrückt werden und so Bewegungen bzw. mechanische Energie übertragen und/oder aufnehmen.

Ferner kann die komplementäre Kolbenanordnung wenigstens eine instrumentenseitige Federvorrichtung umfassen, welche dazu eingerichtet ist, den wenigstens einen komplementären Kolben und/oder den wenigstens einen weiteren komplementären Kolben in einer federbelasteten Position axial vorzuspannen, um eine zuverlässige Kopplung mit der antriebsseitigen Kolbenanordnung zu gewährleisten. In einigen Ausführungsformen können sowohl die Kolbenanordnung als auch die komplementäre Kolbenanordnung wenigstens eine Federvorrichtung umfassen.

Ein weiterer Aspekt der Erfindung betrifft ein medizinisches Robotersystem umfassend eine Schnittstelle der eingangsbezeichneten Art.

Mittels einer solchen Schnittstelle kann ein vorteilhaft weiterentwickeltes medizinisches System bereitgestellt werden. Insbesondere kann eine Installation bzw. Deinstallation medizinischer Instrumente vereinfacht und es können Kosten sowie Stillstandszeiten reduziert werden, da eine durchgängige medizinische Barriere zum Einsatz kommt, die insbesondere nicht komplex geformt ist und/oder nicht speziell eingebettete Adapter zur Übertragung einer Antriebskraft und/oder eines Antriebsdrehmoments aufweist. Hierdurch kann eine zeitaufwändige präzise Platzierung der medizinischen Barriere entfallen. Zudem kann ein Kostenaufwand für jeden medizinischen Einsatz reduziert werden, da eine nur einmalig verwendbare medizinische Barriere möglichst kostengünstig gehalten werden kann. Insbesondere kann eine medizinische Standardbarriere zum Einsatz kommen. Ferner kann durch die erfindungsgemäßen Merkmale eine besonders zuverlässige und funktionsfähige Schnittstelle mit optimierten Eigenschaften bereitgestellt werden, sodass insbesondere vor und/oder während einem medizinischen Eingriff an einem Patienten stets ein steriles Arbeitsfeld gewährleistet bzw. sichergestellt ist.

Die erfindungsgemäßen Vorrichtungen und Systeme sollen hierbei nicht auf die oben beschriebene Anwendung und Ausführungsform beschränkt sein. Insbesondere können diese zu einer Erfüllung einer hierin beschriebenen Funktionsweise eine von einer hierin genannten Anzahl von einzelnen Elementen, Bauteilen und Einheiten abweichende Anzahl aufweisen. Zudem sollen bei den in dieser Offenbarung angegebenen Wertebereichen auch innerhalb der genannten Grenzen liegende Werte als offenbart und als beliebig einsetzbar gelten.

Im Folgenden wird die vorliegende Erfindung anhand der beigefügten Figuren beispielhaft beschrieben. Die Zeichnungen, die Beschreibung und die Ansprüche enthalten zahlreiche Merkmale in Kombination. Der Fachmann wird die Merkmale zweckmäßigerweise auch einzeln betrachten und im Rahmen der Ansprüche sinnvoll in Kombination verwenden.

Falls von einem bestimmten Objekt mehr als ein Exemplar vorhanden ist, ist ggf. nur eines davon in den Figuren und in der Beschreibung mit einem Bezugszeichen versehen. Die Beschreibung dieses Exemplars kann entsprechend auf die anderen Exemplare von dem Objekt übertragen werden. Sind Objekte insbesondere mittels Zahlenwörtern, wie beispielsweise erstes, zweites, drittes Objekt etc. benannt, dienen diese der Benennung und/oder Zuordnung von Objekten. Demnach können beispielsweise ein erstes Objekt und ein drittes Objekt, jedoch kein zweites Objekt umfasst sein. Allerdings könnten anhand von Zahlenwörtern zusätzlich auch eine Anzahl und/oder eine Reihenfolge von Objekten ableitbar sein.

Es zeigen:
- Fig. 1: ein medizinisches Robotersystem mit einer Schnittstelle zur Verbindung einer Antriebseinheit mit einem medizinischen Instrument,
- Fig. 2: eine perspektivische Darstellung der Schnittstelle mit einer antriebsseitigen Kopplungsvorrichtung und einer instrumentenseitigen Kopplungsvorrichtung in einem aneinander angelegten Zustand,
- Fig. 3: eine seitliche Darstellung der antriebsseitigen Kopplungsvorrichtung und der instrumentenseitigen Kopplungsvorrichtung in einem räumlich voneinander getrennten Zustand,
- Fig. 4: eine perspektivische Darstellung der Schnittstelle bei eingelegter medizinischer Barriere,
- Fig. 5: eine perspektivische Darstellung eines Teils der antriebsseitigen Kopplungsvorrichtung bei entferntem Gehäuse,
- Fig. 6: eine Detailansicht einer Ausführungsform einer Kolbenanordnung der antriebsseitigen Kopplungsvorrichtung,
- Fig. 7: eine schematische Längsschnittdarstellung eines Teils der antriebsseitigen Kopplungsvorrichtung,
- Fig. 8: eine perspektivische Darstellung der instrumentenseitigen Kopplungsvorrichtung mit Fokus auf eine instrumentenseitige Kopplungsfläche,
- Fig. 9: eine perspektivische Darstellung der antriebsseitigen Kopplungsvorrichtung mit Fokus auf eine antriebsseitige Kopplungsfläche,
- Fig. 10: eine perspektivische Darstellung der instrumentenseitigen Kopplungsvorrichtung mit daran gekoppelter antriebsseitiger Kolbenanordnung,
- Fig. 11: eine perspektivische Darstellung der Schnittstelle im gekoppelten Zustand,
- Fig. 12: eine Detailansicht der Schnittstelle im gekoppelten Zustand,
- Fig. 13: eine schematische Darstellung einer weiteren Ausführungsform einer Schnittstelle im gekoppelten Zustand,
- Fig. 14: eine Detailansicht einer weiteren Ausführungsform einer Kolbenanordnung,
- Fig. 15: eine schematische Längsschnittdarstellung einer Schnittstelle gemäß einer weiteren Ausführungsform,
- Fig. 16: eine schematische Längsschnittdarstellung der Schnittstelle gemäß Fig. 15 in gekoppelten Zustand,
- Fig. 17: ein Ablaufdiagramm eines Verfahrens zum Verbinden der Kopplungsvorrichtungen unter Zwischenlage der medizinischen Barriere.

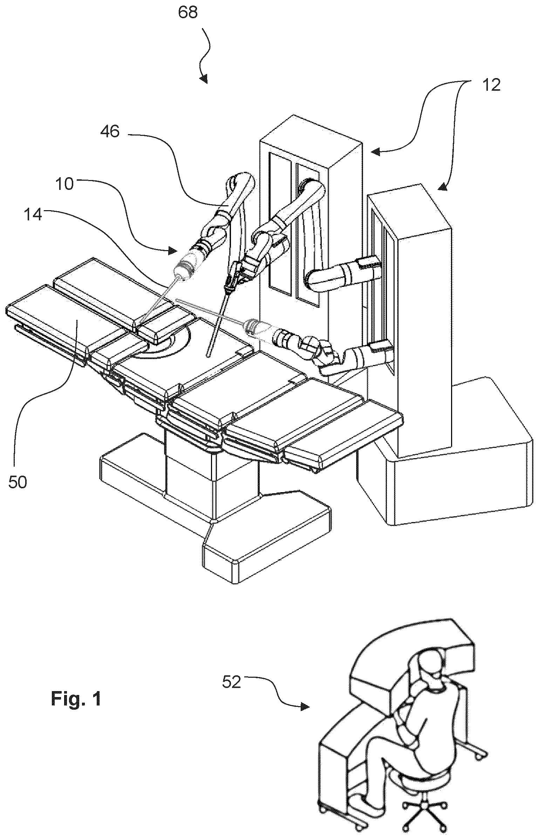

Fig. 1 zeigt ein medizinisches Robotersystem 68. Das Robotersystem 68 weist eine Patientenliege 50 auf. Das Robotersystem weist einen Roboterarm 46 und eine Antriebseinheit 12 auf, wobei der Roboterarm 46 über die Antriebseinheit 12 steuerbar ist. Eine Ansteuerung der Antriebseinheit 12 kann automatisiert, teilautomatisiert und/oder durch medizinisches Personal mittels einer Bedienkonsole 52 erfolgen.

Das Robotersystem weist ein medizinisches Instrument 14 auf, welches an dem Roboterarm 46 angeordnet und durch diesen bewegbar ist. Das medizinische Instrument 14 selbst weist zumindest ein bewegliches Teil auf, welches gegenüber einem weiteren Teil des medizinischen Instruments 14 beweglich ist. Das bewegliche Teil ist durch die Antriebseinheit 12 relativ zu dem weiteren Teil bewegbar. Vorliegend handelt es sich bei dem medizinischen Instrument 14 um eine Zange, wobei auch beliebige andere, dem Fachmann als sinnvoll erscheinende Instrumente denkbar wären. Der Übersichtlichkeit halber sind nicht alle Komponenten des Robotersystems mit Bezugszeichen versehen.

Um sterile Arbeitsbedingungen zu schaffen, ist eine durchgängige medizinische Barriere 24 vorgesehen, welche einen sterilen Bereich um die Patientenliege 50 von der potenziell unsterilen Antriebseinheit 12 und dem Roboterarm 46 trennt. Eine Antriebskraft und/oder ein Antriebsdrehmoment für das bewegliche Teil des medizinische Instrument 14 wird mittels einer Schnittstelle 10 des Robotersystems übertragen. Die Schnittstelle 10 ist in einem Einsatzzustand im Bereich der medizinischen Barriere 24 angeordnet. Die Schnittstelle 10 ist in dem Einsatzzustand durch den Roboterarm 46 gehalten und geführt.

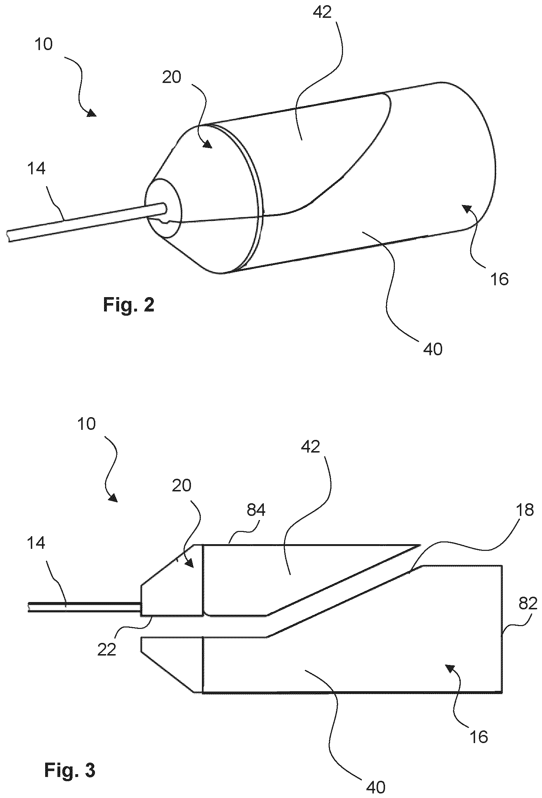

Die Schnittstelle 10 weist eine antriebsseitiges Kopplungsvorrichtung 16 und eine instrumentenseitige Kopplungsvorrichtung 20 auf, welche in Fig. 2 in einer perspektivischen Darstellung in einem aneinander angelegten Zustand gezeigt sind. Fig. 3 zeigt die Kopplungsvorrichtungen 16, 20 in einem voneinander räumlich getrennten Zustand. Ferner weisen die Kopplungsvorrichtungen 16, 20 jeweils ein Gehäuse 82, 84 auf, die verliersicher miteinander verbindbar sind. Die Verbindung der beiden Gehäuse 82, 84 ist lösbar.

Die antriebsseitige Kupplungsvorrichtung 16 weist eine antriebsseitige Kopplungsfläche 18 auf. Die instrumentenseitige Kopplungsvorrichtung 20 umfasst eine instrumentenseitige Kopplungsfläche 22. Die Kopplungsflächen 18, 22 sind zueinander komplementär ausgebildet und erlauben ein formschlüssiges Zusammenfügen der Verbindungselemente 16, 20, wie in Fig. 2 dargestellt.

Die Kopplungsvorrichtungen 16, 20 bzw. deren Gehäuse 82, und 80 bilden zusammengesetzt einen zumindest teilweise kreiszylindrischen und zumindest teilweise kegelstumpfförmigen Körper (vgl. Fig. 2 und 3).

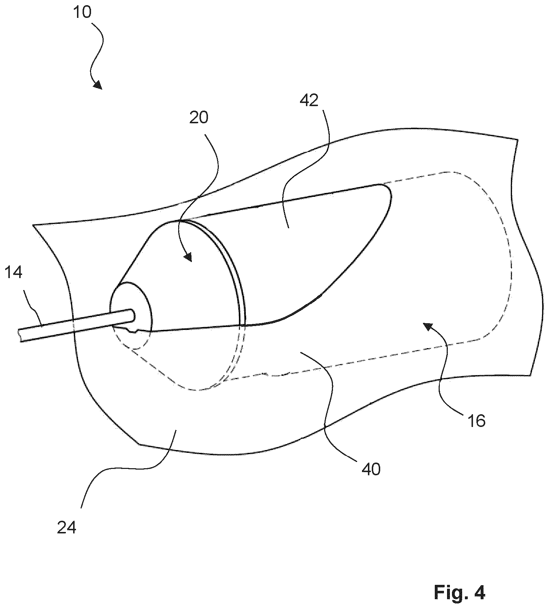

Die Kopplungsflächen 18, 22 sind dazu eingerichtet, die medizinische Barriere 24 einzuklemmen (vgl. Fig. 4). Die Kopplungsvorrichtungen 16, 20 sind über ihre jeweiligen Kopplungsflächen 18, 22 unter Zwischenschaltung der medizinischen Barriere 24 miteinander koppelbar. Bei der medizinischen Barriere 24 handelt es sich um eine durchgängige Kunststofffolie, welche insbesondere frei von Öffnungen ist. In dem Einsatzzustand sind die beiden Kopplungsvorrichtungen 16, 20 mittels der medizinischen Barriere 24 voneinander getrennt. Dabei befindet sich die instrumentenseitige Kopplungsvorrichtung 20 zusammen mit dem medizinischen Instrument 14 in dem Einsatzzustand auf einer sterilen Seite der medizinischen Barriere 24. Die antriebsseitige Kopplungsvorrichtung 16 wird in dem in Figur 4 gezeigten Einsatzzustand von der medizinischen Barriere 24 weitestgehend verdeckt, was durch die gestrichelten Linien angedeutet ist.

Die instrumentenseitige Kopplungsvorrichtung und/oder deren Gehäuse kann beispielsweise über eine hierin nicht dargestellte Klemmschelle und/oder mittels einer sonstigen vorteilhaften, vorzugsweise reversiblen, Befestigungsvorrichtung verliersicher mit der antriebsseitigen Kopplungsvorrichtung und/oder deren Gehäuse zusammengehalten werden.

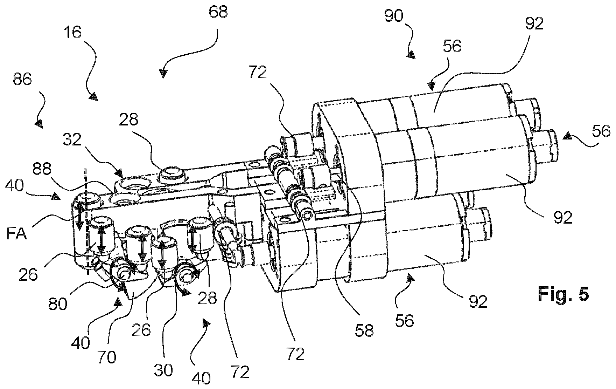

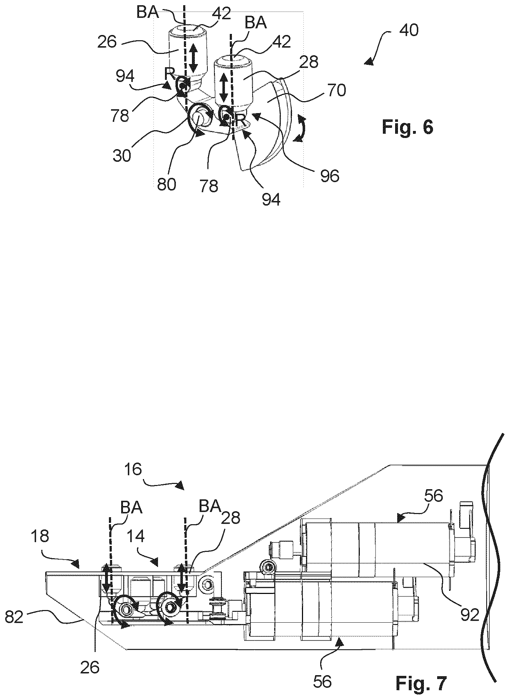

Eine Übertragung einer Antriebskraft und/oder eines Antriebsdrehmoments durch die durchgängige medizinische Barriere 24 erfolgt erfindungsgemäß mechanisch über eine Kolbenanordnung 40. In Fig. 5 ist eine perspektivische Darstellung eines Teils der antriebsseitigen Kopplungsvorrichtung 16 mit insgesamt vier antriebsseitigen Kolbenanordnungen 40 in einem Abschnitt 86 der Kopplungsvorrichtung zu erkennen. Für Darstellungszwecke wurde das Gehäuse 82 der antriebsseitigen Kopplungsvorrichtung 16 ausgeblendet. Fig. 5 zeigt demnach das Innere der antriebsseitigen Kopplungsvorrichtung 16. Jede der vier antriebsseitigen Kolbenanordnungen 40 umfasst jeweils zwei Kolben 26, 28, die über eine Hebelvorrichtung 30 interdependent miteinander gekoppelt sind (vgl. Fig. 6). Die Kolbenanordnungen 40 sind derart positioniert, dass alle Kolben 26, 28 die gleiche räumliche Orientierung jedoch parallel zueinander versetzt aufweisen. Ferner ist eine Zylinderführung 32 zu erkennen. Um die einzelnen Komponenten besser erkennen zu können wurde ein vorderer Teil der Zylinderführung 32 ausgeblendet. Die Zylinderführung 32 weist vorliegend Lochbohrungen 88 auf. Jede der Lochbohrungen 88 ist dazu eingerichtet, einen Kolben 26, 28 entlang einer im wesentlich vertikal verlaufenden Führungsachse FA zu führen und eine Bewegung in horizontaler Richtung zu verhindern. Die Freiheitsgrade sind über Pfeile angedeutet.

In einem Abschnitt 90 weist die in Fig. 5 gezeigte antriebsseitige Kopplungsvorrichtung 16 insgesamt vier Antriebsquellen 56 auf, wobei die vierte nicht sichtbar hinter den drei zu erkennenden Antriebsquellen 56 angeordnet ist. Die Antriebsquellen 56 sind vorliegend als Gleichstrommotoren 92 ausgebildet. Es versteht sich von selbst, dass in anderen Ausführungsformen je nach Anwendungsbereich alternativ oder zusätzlich auch andere Antriebsquellen eingesetzt werden können. Insbesondere kann die wenigstens eine Antriebsquelle 56 zusätzlich oder alternativ wenigstens einen Elektromagneten, wenigstens einen Piezoaktor, wenigstens einen hydraulischen Aktuator und/oder wenigstens einen pneumatischen Aktuator umfassen. Die Antriebsquellen 56 sind parallel zueinander angeordnet und erstrecken sich im Wesentlichen in horizontaler Raumrichtung hin zu den antriebsseitigen Kolbenanordnungen 40.

Die antriebsseitigen Kopplungsvorrichtung 16 weist in der hierin beispielhaft gezeigten Ausführungsform ferner Riemengetriebe auf, die dazu eingerichtet sind, jeweils eine antriebsseitige Kolbenanordnung 40 und/oder eine Hebelvorrichtung 30 anzutreiben. Jede Antriebsquelle 56 ist mit einer jeweiligen antriebsseitigen Kolbenanordnung 40 drehmomentübertragend über ein jeweiliges Riemengetriebe gekoppelt. Jedes Riemengetriebe umfasst eine Antriebsquelle 56 mit einer Antriebswelle 58. Ferner umfasst jedes Riemengetriebe eine Antriebsvorrichtung in Form einer Abtriebsscheibe 70, die auf einer Abtriebsachse 80 angeordnet ist. Die Abtriebsachse 80 ist drehmomentübertragend mit der Hebelvorrichtung 30 gekoppelt. Um ein Drehmoment bzw. eine Kraft von der Antriebsquelle 56 auf die antriebsseitige Kolbenanordnung 40 zu übertragen, umfasst die antriebsseitige Kopplungsvorrichtung 16 wenigstens einen hierin nicht dargestellten Riemen, der eine der Antriebswellen 58 mit einer korrespondierenden Abtriebsscheibe 70 verbindet. Um den Riemen in der richtigen Position zu halten und dessen Bewegung zu führen, um eine effiziente Kraftübertragung zu gewährleisten und den Bauraum effizient bzw. platzsparend auszunutzen, weist die antriebsseitigen Kopplungsvorrichtung 16 ferner Umlenkrollen 72 auf. Alternativ oder zusätzlich können auch andere Getriebearten vorgesehen werden. Insbesondere können auch Schneckengetriebe, Maltesergetriebe, hydraulische Getriebe, pneumatische Getriebe und/oder Zahnradgetriebe eingesetzt werden. Der Übersichtlichkeit halber sind nicht alle Komponenten der antriebsseitigen Kopplungsvorrichtung 16 mit Bezugszeichen versehen.

Fig. 6 zeigt eine Detailansicht einer Ausführungsform einer antriebsseitigen Kolbenanordnung 40 gemäß Fig. 5. Die Kolbenanordnung 40 umfasst die Abtriebsachse 80, entlang der die Hebelvorrichtung 30 und die Abtriebsscheibe 70 angeordnet sind. Die Hebelvorrichtung 30 weist zwei Hebelenden 94 bzw. Vorsprünge auf, die sich in entgegengesetzte Raumrichtungen radial von der Abtriebsachse 80 weg erstrecken. Auf jedem der beiden Hebelenden 94, ist einer der Kolben 26, 28 angeordnet, deren Stirnflächen 42 von der Hebelvorrichtung 30 abgewandt sind. An deren Unterseiten 96 weisen die Kolben 26, 28 Nocken 78 auf, über die sie die Hebelenden 94 kontaktieren. Die Nocken 78 sind drehbar mit der Hebelvorrichtung 30 verbunden, sodass die Kolben 26, 28 im Falle einer Drehbewegung der Hebelvorrichtung 30 um eine Rotationsachse R, die sich in die Zeichenebene hinein erstreckt, stets dieselbe Orientierung bzw. Ausrichtung beibehalten. Über die Hebelvorrichtung 30 stehen die beiden Kolben 26 in einer Abhängigkeit. In anderen Worten können die beiden Kolben 26, 28 nicht unabhängig voneinander bewegt werden. Jedoch sind die Bewegungsrichtungen der Kolben 26, 28 aufgrund der Ausgestaltung der Hebelvorrichtung 30 stets konträr bzw. gegensätzlich.

Figur 7 zeigt eine schematische Längsschnittdarstellung der antriebsseitigen Kopplungsvorrichtung 16. Zu erkennen ist, die kompakte und platzsparende Anordnung der antriebsseitigen Kolbenanordnungen 40, Antriebsquellen 56 und Getrieben in einem Gehäuse 82. Zu erkennen ist ferner, dass die Bezugsachsen BA entlang derer die Kolben bewegbar sind, senkrecht zu der antriebsseitigen Kopplungsfläche 18 verlaufen. In der gezeigten Ausführungsform sind die Kolben 26, 28 dazu eingerichtet, sich zumindest abschnittsweise aus der antriebsseitigen Kopplungsfläche 18 herauszubewegen.

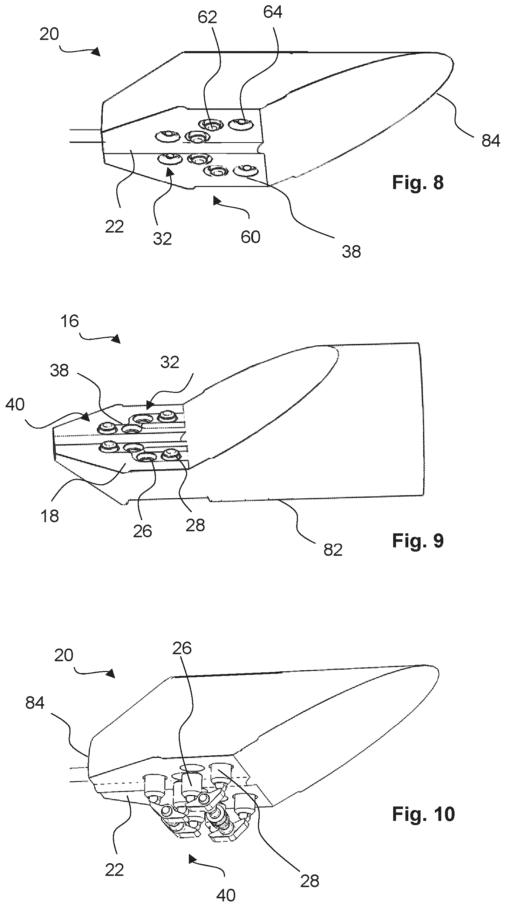

Die Abbildungen 8 und 9 zeigen eine perspektivische Darstellung der instrumentenseitigen Kopplungsvorrichtung 20 bzw. der antriebsseitigen Kopplungsvorrichtung 16 mit Fokus auf die jeweiligen Kopplungsflächen 18, 22. Zu erkennen ist, dass die instrumentenseitige Kopplungsvorrichtung 20 wenigstens eine zu der wenigstens einen antriebsseitigen Kolbenanordnung 40 komplementäre instrumentenseitige Kolbenanordnung 60 umfasst, die dazu eingerichtet ist, Bewegungen der wenigstens einen antriebsseitigen Kolbenanordnung 40 aufzunehmen und/oder Bewegungen auf die wenigstens eine antriebsseitige Kolbenanordnung 40 zu übertragen. Die komplementäre instrumentenseitige Kolbenanordnung 60 umfasst wenigstens einen komplementären instrumentenseitigen und wenigstens einen weiteren komplementären instrumentenseitigen Kolben 62, 64.

Wie nachfolgend noch weiter ausgeführt wird, müssen die komplementären Kolben 62, 64 nicht zwangsweise über eine Hebelvorrichtung 30 miteinander gekoppelt sein.

Auch die instrumentenseitige Kopplungsvorrichtung 20 weist Zylinderführungen 32 auf, die komplementär zu den antriebsseitigen Zylinderführungen 32 ausgebildet sind. Die Zylinderführungen 32 der instrumentenseitigen Kopplungsvorrichtung 20 und der antriebsseitigen Kopplungsvorrichtung 16 sind insbesondere derart ausgebildet bzw. angeordnet, dass diese, in einem Zustand der Schnittstelle 10, in dem die antriebsseitige Kopplungsvorrichtung 16 mit der instrumentenseitigen Kopplungsvorrichtung 20 gekoppelt ist, deckungsgleich sind, sodass ein Kolben 26, 28 der antriebsseitigen Kopplungsvorrichtung im gekoppelten Zustand der Schnittstelle 10 zumindest abschnittsweise in die instrumentenseitige komplementäre Zylinderführung 32 eindringen kann. Vorteilhafterweise sind alle Kanten 38 der Kopplungsvorrichtung 16, 20 und insbesondere alle Kanten 38 der Kopplungsflächen 18, 22, und/oder der Kolbenanordnungen abgerundet. So kann das Risiko einer Beschädigung und einer damit einhergehenden Kontamination des Arbeitsfelds gesenkt werden.

Eine funktionsgemäße Kopplung der antriebsseitigen Kolbenanordnungen 40 mit der komplementären instrumentenseitigen Kolbenanordnung 60 ist beispielhaft in Fig. 10 gezeigt. Der Übersichtlichkeit halber wurden bei dieser Darstellung die Antriebsquelle 56, die Getriebeanordnung sowie das Gehäuse 82 der antriebsseitigen Kopplungsvorrichtung 16 ausgeblendet. Die medizinische Barriere 24 ist ebenfalls nicht dargestellt.

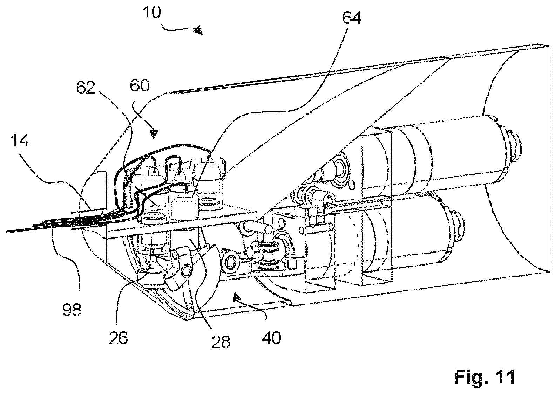

Fig. 11 zeigt eine perspektivische Darstellung einer Schnittstelle 10 im gekoppelten Zustand. Die antriebsseitigen Kolben 26, 28 stehen in physischem Kontakt zu den komplementären instrumentenseitigen Kolben 62, 64. In der gezeigten Ausführungsform sind die komplementären instrumentenseitigen Kolben 62, 64 ferner mit Zugkabeln 98 gekoppelt, um Bewegungen auf ein an die Zugkabel 98 angeschlossenes medizinisches Instrument 14 zu bewegen. Durch Ziehen oder Drücken der Zugkabel 98 können die Position eines medizinischen Instruments 14 und/oder dessen Aktuatorik präzise und/oder remote gesteuert werden. In weiteren hierin nicht gezeigten Ausführungsformen kann die Schnittstelle 10 zusätzliche oder alternative für einen spezifischen Anwendungsfall vorteilhafte Kopplungsmechanismen zwischen den komplementären instrumentenseitigen Kolben 62, 64 und dem medizinischen Instrument 14 bzw. dessen Effektoren vorgesehen werden. Die medizinische Barriere 24 ist in dieser Darstellung ebenfalls nicht dargestellt.

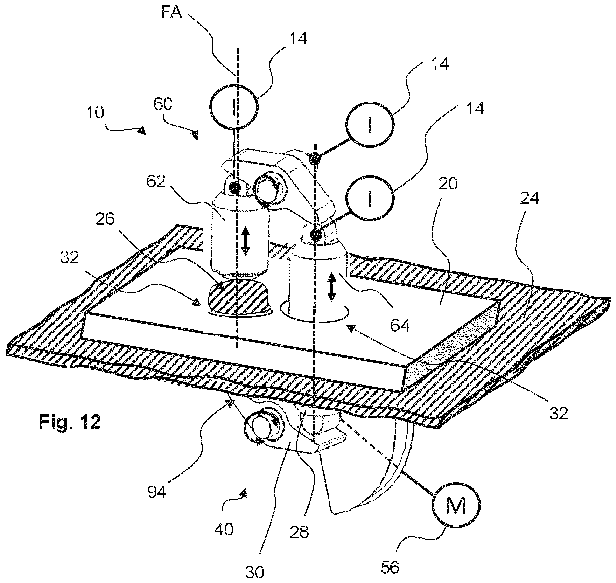

In Fig. 12 ist eine Detailansicht der Schnittstelle 10 im gekoppelten Zustand gezeigt. Zu erkennen ist die antriebsseitige Kolbenanordnung 40, die unter Zwischenschaltung einer medizinischen Barriere 24 mit einer komplementären instrumentenseitigen Kolbenanordnung 60 mechanisch gekoppelt ist. Durch eine Aktuierung der Hebelvorrichtung 30 wird der linke antriebsseitige Kolben 26 von dem linken Hebelende 94 nach oben gedrückt. Dabei wird der Kolben 26 durch eine Zylinderführung 32 entlang einer Führungsachse FA bewegt. Ferner drückt der Kolben 26 in einen Abschnitt der medizinischen Barriere 24, der im gekoppelten Zustand der Schnittstelle 10 die Zylinderführung 32 bedeckt, in die instrumentenseitige Kopplungsvorrichtung 20 bzw. dessen komplementäre Zylinderführung 32. Da die antriebsseitigen Kolben 26, 28 mit den instrumentenseitigen Kolben 62, 64 mechanisch miteinander gekoppelt sind, übt der hochgedrückte linke antriebsseitige Kolben 26 bzw. in vorliegenden Fall die dazwischengeschaltete medizinische Barriere 24 eine Kraft auf den instrumentenseitigen komplementären linken Kolben 60 aus. Da die resultierende Kraft ausgehend von der antriebsseitigen Hebelvorrichtung 30 sowohl auf den antriebsseitigen Kolben 26 als auch auf den komplementären instrumentenseitigen Kolben 60 wirkt, werden beide Kolben 26, 60 in dieselbe Richtung bewegt. Der rechte instrumentenseitige Kolben 62 ist über eine Hebelvorrichtung 30 interdependent mit dem linken instrumentenseitigen Kolben 64 gekoppelt, sodass dieser nach unten gedrückt wird. Der rechte Kolben 62 drückt die medizinische Barriere 24 in eine, in der Darstellung von der medizinischen Barriere 24 verdeckte, antriebsseitige Zylinderführung 32 hinein. Dabei drückt der rechte instrumentenseitige Kolben 62 auf den rechten antriebsseitigen Kolben 28, sodass dieser ebenfalls nach unten gedrückt wird. Es versteht sich von selbst, dass die Richtung, in der die Hebelvorrichtung 30 aktuierbar ist, variieren kann. Die auf die instrumentenseitigen Kolben 62, 64 übertragene mechanische Kraft und/oder Bewegung kann in der vorliegend gezeigten Ausführungsform anschließend über ein in dieser Darstellung nicht gezeigtes Getriebe auf den Endeffektor des medizinischen Instruments 14 übertragen werden, sodass dieser eine entsprechende Bewegung ausführt. In alternativen Ausführungsformen können die instrumentenseitigen Kolben 62, 64 unmittelbar über beispielsweise Zugkabel 98 mit dem Endeffektor des medizinischen Instruments 14 gekoppelt sein und auf diese Weise mechanische Energie übertragen.

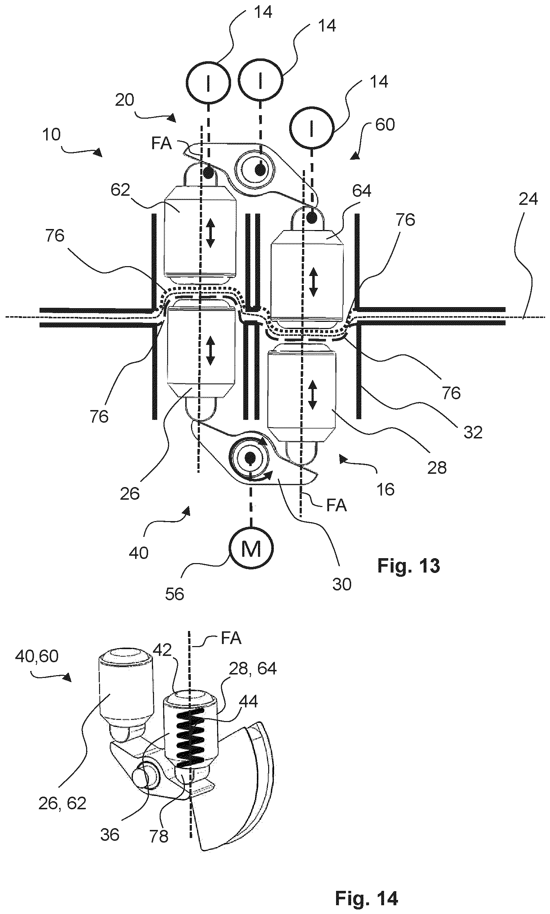

In Fig. 13 ist eine schematische Darstellung einer weiteren Ausführungsform einer Schnittstelle 10 im gekoppelten Zustand gezeigt. Gemäß der darin gezeigten Ausführungsform umfasst die Schnittstelle 10 eine antriebsseitige Kopplungsvorrichtung 16 sowie eine komplementäre instrumentenseitige Kopplungsvorrichtung 20 mit elastischen Membranen 76. Die elastischen Membranen 76 sind dazu eingerichtet, zumindest abschnittsweise Stirnflächen 42 eines Kolbens 26, 28, 62, 64, zu bedecken. Eine derartige Ausgestaltung senkt das Risiko einer Beschädigung einer medizinischen Barriere 20, da sie verhindert, dass die medizinische Barriere 20 etwa zwischen dem Kolben 26, 28, 62, 64 und dessen Zylinderführung 32 eingeklemmt und möglicherweise beschädigt wird. Aufgrund der Elastizität der Membranen 76 kann diese mechanisch ausgewölbt und/oder eingedrückt werden und so Bewegungen bzw. mechanische Energie übertragen und/oder aufnehmen. In alternativen oder zusätzlichen hier nicht gezeigten Ausführungsformen können die Membranen beispielsweise hydraulisch und/oder pneumatisch verformt und/oder ausgewölbt werden.

Fig. 14 zeigt eine Detailansicht einer weiteren Ausführungsform einer Kolbenanordnung 40, 60. Die hierin gezeigte Ausführungsform kann sowohl antriebsseitig als auch instrumentenseitig Anwendung finden. Die hierin gezeigte Ausführungsform der Kolbenanordnung 40, 60 umfasst eine Federvorrichtung 44, die dazu eingerichtet ist, einen wenigstens einen komplementären instrumentenseitigen Kolben 62 und/oder einen wenigstens einen weiteren komplementären instrumentenseitigen Kolben 64 bzw. einen wenigstens einen antriebsseitigen Kolben 26 und/oder einen wenigstens einen weiteren antriebsseitigen Kolben 28 in einer federbelasteten Position axial vorzuspannen, um stets eine zuverlässige Kopplung mit der antriebsseitigen Kolbenanordnung 40 zu gewährleisten. In der hierin gezeigten Ausführungsform ist die Federvorrichtung 44 zwischen einem Nocken 78, der eine Hebelvorrichtung kontaktiert und einer Stirnfläche 42 des Kolbens 28, 64 angeordnet. Die Stirnfläche 42 des Kolbens 26, 62 ist dabei mit der Federvorrichtung 44 verbunden. Ferner ist die Stirnfläche 42 relativ zu einer Kolbenwand 36 entlang einer Führungsachse FA bewegbar. An dieser Stelle sei darauf hingewiesen, dass die gezeigte Ausführungsform lediglich beispielhaft ist. Die Federvorrichtung 44 muss nicht zwingend, wie in der Fig. 13 dargestellt, in den Kolben 26, 28, 62, 64 integriert sein, sie kann alternativ oder zusätzlich auch vorteilhaft außerhalb des Kolbens 26, 28, 62, 64, beispielsweise zwischen einem Nocken 78 und einer Unterseite 96 des Kolbens 26, 28, 62, 64 angeordnet sein. In einigen Ausführungsform kann der Kolben 26, 28, 62, 64 selbst zumindest zu einem Großteil als eine Federvorrichtung 44 ausgebildet sein.

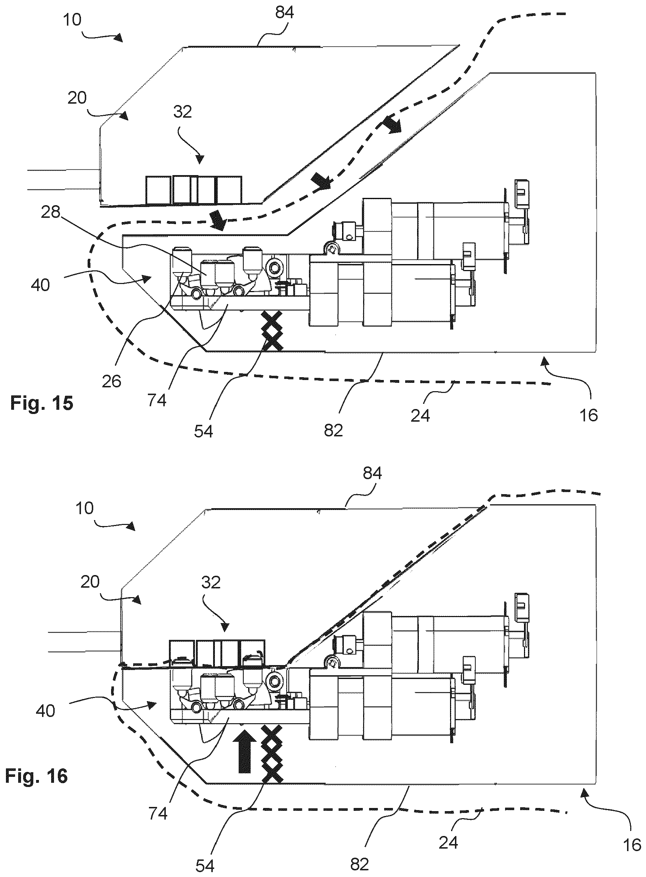

Eine Weiterbildung der vorangehend beschriebenen Schnittstellen 10 ist in den Fig. 15 und 16 dargestellt. Die Fig. 15 und 16 zeigen eine schematische Längsschnittdarstellung einer Schnittstelle 10, bei der eine antriebsseitige Kopplungsvorrichtung 16 wenigstens eine Hubvorrichtung 54 umfasst, die dazu eingerichtet ist, zumindest wenigstens eine antriebsseitige Kolbenanordnung 40 und/oder wenigstens eine antriebsseitige Kopplungsvorrichtung 16 anzuheben, um die antriebsseitige Kopplungsvorrichtung 16 funktionsfähig mit einer instrumentenseitigen Kopplungsvorrichtung 20 zu koppeln. Ferner erleichtert eine derartige Hubvorrichtung 54 den ordnungsgemäßen Kopplungsvorgang. In der gezeigten Ausführungsform ist die Hubvorrichtung 54 unter der antriebsseitigen Kolbenanordnung 40 angeordnet. Die Hubvorrichtung 54 ist dabei fest mit einem Gehäuse 82 der antriebsseitigen Kopplungsvorrichtung 16 und einem Gestänge 74, auf dem die antriebsseitigen Kolbenanordnung 40 bzw. ein Getriebe positioniert bzw. angebracht ist, verbunden. Es versteht sich von selbst, dass die Position der Hubvorrichtung 54 keineswegs auf die hierin gezeigte Ausführungsform beschränkt ist. Ferner ist die antriebsseitige Kolbenanordnung 40 in einem nicht gekoppelten Zustand derart in dem Gehäuse 82 der antriebsseitigen Kopplungsvorrichtung 16 positioniert, dass kein Kolben 26, 28 der Kopplungsvorrichtung 16 aus einer antriebsseitigen Kopplungsfläche 18 herausragt. In diesem Ruhe- oder Transportzustand ist die empfindliche Mechanik innerhalb des Gehäuses 82 der antriebsseitigen Kopplungsvorrichtung 16 weitestgehend vor äußeren Einflüssen geschützt.

Eine Schnittstelle 10 gemäß dieser Ausführungsform kann dazu eingerichtet sein, zu detektieren, ob die antriebsseitige Kopplungsfläche 18 ordnungsgemäß mit einer instrumentenseitigen Kopplungsfläche 22 verbunden wurde. Ist dies der Fall, kann die Hubvorrichtung 54 aktiviert werden und die antriebsseitige Kolbenanordnung 40 in eine Betriebsposition anheben. Durch den Anhebevorgang kann zumindest ein Abschnitt des wenigstens einen und/oder des wenigstens einen weiteren antriebsseitigen Kolbens 26, 28 in die instrumentenseitige Kopplungsvorrichtung 20, insbesondere in eine komplementäre instrumentenseitige Zylinderführung 32 hineingedrückt werden. Zusätzlich oder alternativ zu der hierin beschriebenen Ausführungsform kann auch die instrumentenseitige Kopplungsvorrichtung 20 eine Hubvorrichtung 54 umfassen, die entsprechend dazu eingerichtet ist, die komplementäre instrumentenseitige Kolbenanordnung 60 in die antriebsseitige Kopplungsvorrichtung 16, insbesondere in die antriebsseitige Zylinderführung 32 hineinzudrücken.

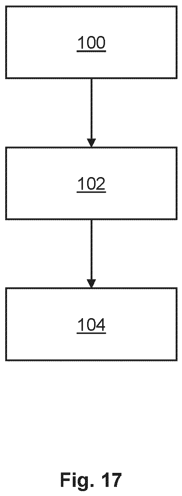

Fig. 17 zeigt ein Ablaufdiagramm eines Verfahrens zum Verbinden der Kopplungsvorrichtungen 16, 20 unter Zwischenlage der medizinischen Barriere 24. In einem Schritt 100 wird die erste Kopplungsfläche 18 und das Gehäuse 82 der antriebsseitigen Verbindungselements 16 zumindest abschnittsweise mit der medizinischen Barriere 24 bedeckt. In einem Schritt 102 wird das instrumentenseitige Verbindungselement 20 über seine instrumentenseitige Kopplungsfläche 22 unter Zwischenlage der medizinischen Barriere 24 mit der ersten Kopplungsfläche 18 verbunden. In einem Schritt 104 wird schließlich die Hubvorrichtung 54 betätigt und die Schnittstelle 10 in einen Benutzungszustand überführt.

### Bezugszeichenliste

- 10: Schnittstelle
- 12: Antriebseinheit
- 14: medizinisches Instrument
- 16: antriebsseitige Kopplungsvorrichtung
- 18: antriebsseitige Kopplungsfläche
- 20: instrumentenseitiges Kopplungsvorrichtung
- 22: instrumentenseitige Kopplungsfläche
- 24: medizinische Barriere
- 26: Kolben
- 28: weiterer Kolben
- 30: Hebelvorrichtung
- 32: Zylinderführung
- 34: Führungshülse
- 36: Kolbenwand
- 38: Kante
- 40: antriebsseitige Kolbenanordnung
- 42: Stirnfläche
- 44: Federvorrichtung
- 46: Roboterarm
- 48: Rastausnehmung
- 50: Patientenliege
- 52: Bedienkonsole
- 54: Hubvorrichtung
- 56: Antriebsquelle
- 58: Antriebswelle
- 60: komplementäre instrumentenseitige Kolbenanordnung
- 62: komplementärer Instrumentenseitiger Kolben
- 64: komplementärer instrumentenseitiger weiterer Kolben
- 68: Medizinisches System
- 70: Abtriebsscheibe
- 72: Umlenkrolle
- 74: Gestänge
- 76: Membran
- 78: Nocken
- 80: Abtriebsachse
- 82: antriebsseitiges Gehäuse
- 84: instrumentenseitiges Gehäuse
- 86: proximaler Abschnitt
- 88: Lochbohrung
- 90: distaler Abschnitt
- 92: Gleichstrommotor
- 94: Hebelende
- 96: Unterseite
- 98: Zugkabel
- 100: Schritt
- 102: Schritt
- 104: Schritt
- BA: Bezugsachse
- FA: Führungsachse
- M: Motor

## Patentansprüche

1. Schnittstelle (10) zur Verbindung einer Antriebseinheit (12) mit einem medizinischen Instrument (14) umfassend:
- wenigstens eine antriebsseitige Kopplungsvorrichtung (16) mit wenigstens einer antriebsseitigen Kopplungsfläche (18), und
- wenigstens eine zu der antriebsseitigen Kopplungsvorrichtung (16) komplementäre instrumentenseitigen Kopplungsvorrichtung (20) mit wenigstens einer instrumentenseitigen Kopplungsfläche (22),
wobei die antriebsseitige und die instrumentenseitige Kopplungsvorrichtung (16, 20) unter Zwischenschaltung einer durchgängigen medizinischen Barriere (24) mechanisch miteinander koppelbar sind, und
wobei zumindest die antriebsseitige Kopplungsvorrichtung (16) wenigstens eine antriebsseitige Kolbenanordnung (40) mit wenigstens einem Kolben (26) umfasst, welcher dazu eingerichtet ist, alternierend in entgegengesetzten Richtungen bewegt zu werden.

2. Schnittstelle (10) nach Anspruch 1,
wobei die alternierende Bewegung entlang einer Bezugsachse (BA) erfolgt, welche schräg oder senkrecht zu der antriebsseitigen Kopplungsfläche (18) verläuft.

3. Schnittstelle (10) nach einem der vorherigen Ansprüche,
wobei die antriebsseitige Kolbenanordnung (40) wenigstens einen weiteren Kolben (28) umfasst, welcher mit dem wenigstens einen Kolben (26) interdependent gekoppelt ist.

4. Schnittstelle (10) nach Anspruch 2, weiter umfassend:
wenigstens eine Hebelvorrichtung (30), wobei der wenigstens eine Kolben (26) zusammen mit dem wenigstens einen weiteren Kolben (28) über die wenigstens eine Hebelvorrichtung (30) aktuierbar ist.

5. Schnittstelle (10) nach einem der vorherigen Ansprüche,
wobei die wenigstens eine antriebsseitige Kolbenanordnung (40) wenigstens eine Zylinderführung (32) umfasst, welche dazu eingerichtet ist, den wenigstens einen Kolben (26) und/oder den wenigstens einen weiteren Kolben (28) während einer Bewegung entlang einer Führungsachse (FA) zu führen.

6. Schnittstelle (10) nach Anspruch 5,
wobei jede Zylinderführung (32) wenigstens eine Führungshülse (34) umfasst.

7. Schnittstelle (10) nach Anspruch 5 oder 6,
wobei die wenigstens eine Zylinderführung (32) wenigstens eine Kante (38) aufweist, welche zumindest abschnittsweise, bevorzugt vollständig, abgerundet ist.

8. Schnittstelle (10) nach einem der vorherigen Ansprüche,
wobei der wenigstens eine Kolben (26) und/oder der wenigstens eine weitere Kolben (28) wenigstens eine Stirnfläche (42) aufweist, dessen Kante (38) zumindest abschnittsweise, bevorzugt vollständig abgerundet ist.

9. Schnittstelle (10) nach einem der vorherigen Ansprüche,
wobei die antriebsseitige Kolbenanordnung (40) wenigstens eine Federvorrichtung (44) umfasst, welche dazu eingerichtet ist, den wenigstens einen Kolben (26) und/oder den wenigstens einen weiteren Kolben (28) in einer federbelasteten Position axial vorzuspannen, um eine zuverlässige Bewegungsübertragung auf das medizinische Instrument (14) zu gewährleisten.

10. Schnittstelle (10) nach einem der vorherigen Ansprüche,
umfassend wenigstens eine Hubvorrichtung (54), welche dazu eingerichtet ist, zumindest die wenigstens eine antriebsseitige Kolbenanordnung (40) und/oder die antriebsseitige Kopplungsvorrichtung (16) anzuheben, um die antriebsseitige Kopplungsvorrichtung (16) funktionsfähig mit der instrumentenseitigen Kopplungsvorrichtung (20) zu koppeln.

11. Schnittstelle (10) nach einem der vorherigen Ansprüche,
wobei die antriebsseitige Kopplungsvorrichtung (16) eine Mehrzahl an antriebsseitigen Kolbenanordnungen (40) umfasst.

12. Schnittstelle (10) nach Anspruch 11,
wobei jeder antriebsseitigen Kolbenanordnung (40) ein Freiheitsgrad des medizinischen Instruments (14) zugeordnet ist.

13. Schnittstelle (10) zumindest nach Anspruch 4,
wobei die antriebsseitige Kopplungsvorrichtung (16) wenigstens eine Antriebsquelle (56) umfasst, welche dazu eingerichtet ist, die Hebelvorrichtung (30) anzutreiben.

14. Schnittstelle (10) nach Anspruch 13,
wobei die Antriebsquelle (56) wenigstens eine Antriebswelle (58), wenigstens ein Zahnradgetriebe, wenigstens einen Riemenantrieb, wenigstens einen Seiltrieb, wenigstens einen Kettenantrieb und/oder wenigstens ein Zahnstangengetriebe umfasst.

15. Schnittstelle (10) nach Anspruch 13 oder 14,
wobei die Antriebsquelle (56) wenigstens einen Elektromagneten, wenigstens einen Piezoaktor, wenigstens einen hydraulischen Aktuator und/oder wenigstens einen pneumatischen Aktuator umfasst.

16. Schnittstelle (10) nach einem der vorherigen Ansprüche,
wobei die wenigstens eine instrumentenseitige Kopplungsvorrichtung (20) wenigstens eine zu der wenigstens einen antriebsseitigen Kolbenanordnung (40) komplementäre instrumentenseitige Kolbenanordnung (60) umfasst, die dazu eingerichtet ist, Bewegungen der wenigstens einen antriebsseitigen Kolbenanordnung (40) aufzunehmen und/oder Bewegungen auf die wenigstens eine antriebsseitigen Kolbenanordnung (40) zu übertragen.

17. Schnittstelle (10) nach Anspruch 16,
wobei die komplementäre instrumentenseitige Kolbenanordnung (60) wenigstens einen komplementären instrumentenseitigen und wenigstens einen weiteren komplementären instrumentenseitigen Kolben umfasst.

18. Schnittstelle (10) nach Anspruch 16 oder 17,
wobei die wenigstens eine komplementäre instrumentenseitige Kolbenanordnung wenigstens eine Kante (38) aufweist, welche zumindest abschnittsweise, bevorzugt vollständig, abgerundet ist.

19. Schnittstelle (10) nach Anspruch 17 oder 18,
wobei die komplementäre instrumentenseitige Kolbenanordnung (60) wenigstens eine instrumentenseitige Federvorrichtung (44) umfasst, welche dazu eingerichtet ist, den wenigstens einen komplementären instrumentenseitigen Kolben (62) und/oder den wenigstens einen weiteren komplementären instrumentenseitigen Kolben (64) in einer federbelasteten Position axial vorzuspannen, um eine zuverlässige Kopplung mit dem der antriebsseitigen Kolbenanordnung (40) zu gewährleisten.

20. Medizinisches Robotersystem (68) umfassend eine Schnittstelle (10) nach einem der vorherigen Ansprüche.
